# EUROPEAN PATENT SPECIFICATION

(11) **EP 0 649 902 B1**
(45) Date of publication and mention of the grant of the patent: **22.01.2003**
(21) Application number: 94116646.4
(22) Date of filing: 21.10.1994
(51) Int. Cl.: C12N 15/00, A01K 67/027, G01N 33/50

(54) **Method for screening of prophylactic and therapeutic agents for IgE-related diseases**
Verfahren zum Screenen von Agenzien zur Prophylaxis oder Therapie von mit IgE zusammenhängenden Krankheiten
Procédé pour le criblage des agents prophylactiques et thérapeutiques des affections liées aux IgE

(30) Priority: 22.10.1993 JP 26480293
(43) Date of publication of application: 26.04.1995
(73) Proprietor: Ra, Chisei, Chiba-shi, Chiba 262 (JP)
(72) Inventor: Ra, Chisei, Hanamigawa-ku, Chiba-shi, Chiba (JP); Naito, Koji, c/o The Green Cross Corporation, Hirakata-shi, Osaka (JP); Hirama, Minoru, c/o The Green Cross Corporation, Hirakata-shi, Osaka (JP); Okumura, Ko, Chiba-shi, Chiba (JP)
(74) Representative: Hansen, Bernd, Dr. Dipl.-Chem.

(56) References cited:
- JOURNAL OF IMMUNOLOGY., vol.153, no.8, 15 October 1994, BALTIMORE US pages 3804 - 3810 PESTEL, J. ET AL. 'Human IgG in SCID mice reconstituted with peripheral blood mononuclear cells from Dermatophagoides pteronyssinus-sensitive patients'
- CELLULAR IMMUNOLOGY, vol.151, no.2, 15 October 1993 pages 241 - 256 KILCHHERR, E. ET AL. 'Regulation of human IgE response in hu-PBL-SCID mice'
- JOURNAL OF IMMUNOLOGY., vol.148, no.4, 15 February 1992, BALTIMORE US pages 1065 - 1071 CARLSSON, R. ET AL. 'Human peripheral blood lymphocytes transplanted into SCID mice constitute an in vivo culture system exhibiting several parameters found in a normal humoral immune response and are a source of immunocytes for the production of human monoclonal antibodies'
- ROITT I.M. ET AL: 'IMMUNOLOGY', 1982, GOWER MEDICAL PUBLISHING, LONDON
- PASSMORE R. ET AL: 'A COMPANION TO MEDICAL STUDIES', 1970, BLACKWELL SCIENTIFIC PUBLICATIONS, OXFORD, GB

## Description

### FIELD OF THE INVENTION

The present invention relates to a method of screening prophylactic and therapeutic agents for IgE-related diseases using the same.

### BACKGROUND OF THE INVENTION

Recently, as the mechanism of the allergic reaction has been elucidated, attention is given to the role of IgE and development of various antiallergic drugs has been vigorously conducted with targeting for IgE. In the development of the antiallergic drug, a screening of candidate substances is of importance.

One approach to this end comprises inducing an IgE-mediated allergic reaction and checking to see how far this reaction is inhibited. Such approach includes a method comprising the use of animals subjected to passive immunization with purified IgE or animals subjected to active immunization with an antigen as model animals. Both methods are useful but not suited for inducing an allergic reaction repeatedly for investigating the inhibitory effect of test substances.

Thus, in the former method, IgE must be repeatedly administered in order that the allergic reaction may be induced in repetition, with the result that a drug screening cannot be expediently carried out.

The latter method is also disadvantageous in that even though an allergic reaction can be repeatedly induced, not only the antigen-specific IgE but also other antibodies such as the reaction-interfering antigen-specific IgG etc. are produced in the body so that the inhibitory effect on IgE-mediated allergic reaction cannot be accurately estimated.

E. Kilchherr et al describe the regulation of human IgE response in hu-PBL-SCID mice (Cellular Immunology 151, 241-256(1993)).

### SUMMARY OF THE INVENTION

An object of the present invention is to solve the above-described problems and to provide an animal model which enables repeated .observation of IgE-mediated allergic reaction and expedient screening of prophylactic and therapeutic agents for IgE-related diseases and a method of screening prophylactic and therapeutic agents for IgE-related diseases using the same.

The above-mentioned object can be achieved by a method of screening prophylactic and therapeutic agents for IgE-related diseases using a rodent model which is constructed by transplanting an IgE-producing vegetative cell in a non-rejecting rodent to induce IgE-mediated allergic reaction caused by the transplanted cells.

### BRIEF DESCRIPTION OF THE DRAWINGS

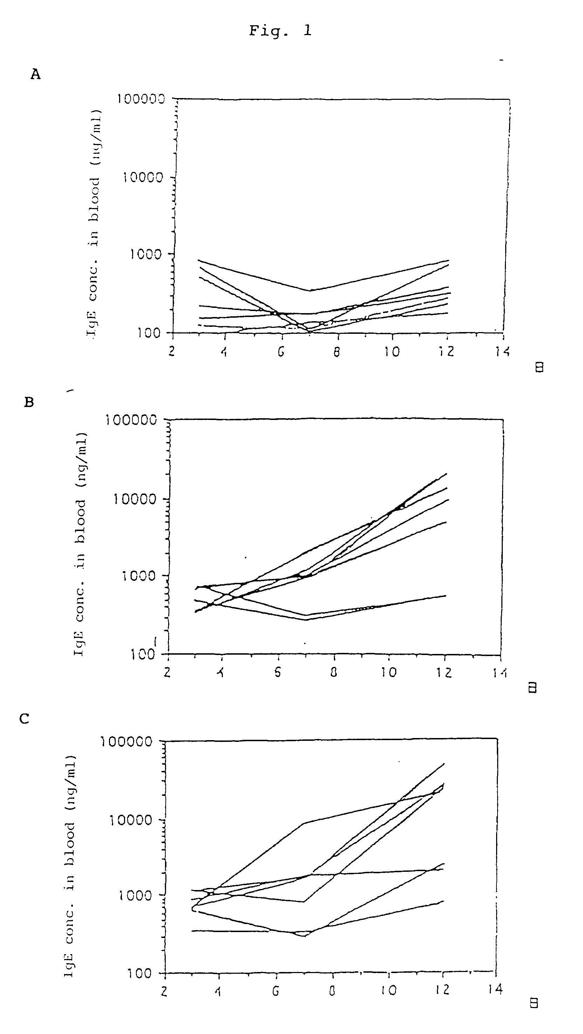

Fig. 1 graphically shows the time course of anti-trinitrophenyl (TNP)•IgE level in blood collected from each mouse into which anti-TNP•IgE producing hybridoma IGELa2 has been transplanted or not transplanted. In Fig. 1, A shows the results of untransplanted group, B shows the results of 10⁶ IGELa2-transplanted group and C shows the results of 10⁷ IGELa2-transplanted group.

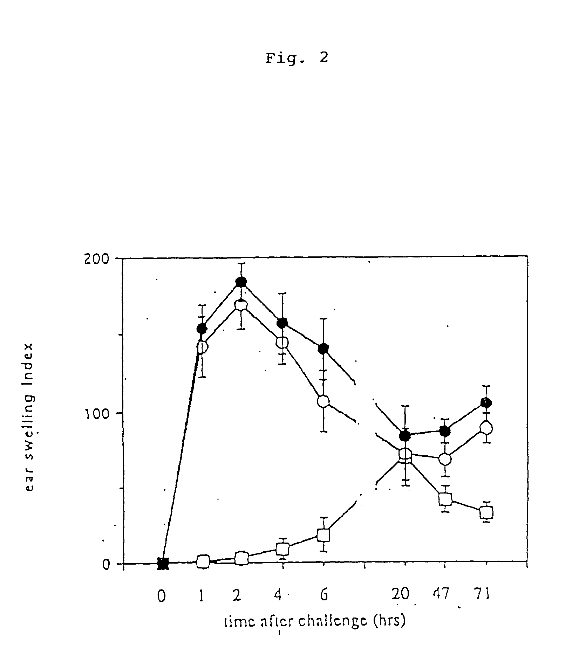

Fig. 2 shows the results of ear swelling in antigen-challenged mice into which anti-TNP•IgE-producing hybridoma IGELa2 has been transplanted or not transplanted. In Fig. 2, - □ - stands for control group (n=7), - o - for 10⁶ IGELa2-transplanted group (n=7) and - ● - for 10⁷ IGELa2-transplanted group (n=6 or 7).

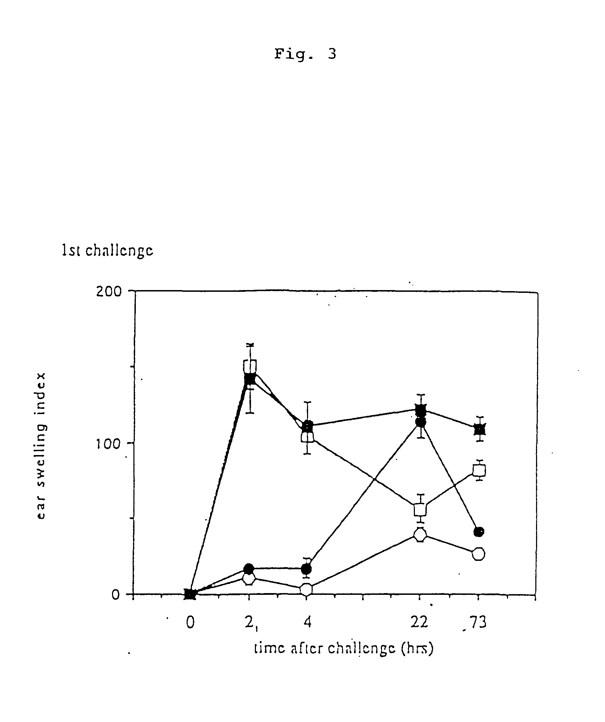

Fig. 3 shows the results of ear swelling in the first antigen-challenged mice into which anti-TNP•IgE-producing hybridoma IGELa2 has been transplanted or not transplanted. In Fig. 3, - o - stands for 0.4% picryl chloride (PC)-challenged untransplanted group (n=12), - ● - for 4% PC-challenged untransplanted group (n=12), - □ - for 0.4% PC-challenged IGELa2-transplanted group (n=12), - ■ - for 4% PC-challenged IGELa2-transplanted group (n=11).

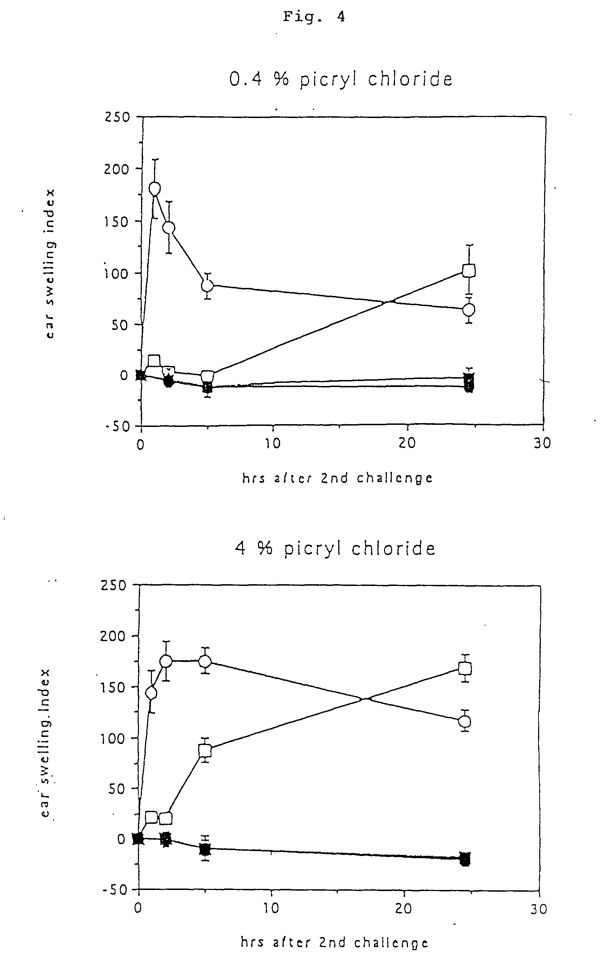

Fig. 4 shows the results of ear swelling in the second antigen-challenged mice into which anti-TNP•IgE-producing hybridoma IGELa2 has been transplanted or not transplanted. The mice used in the experiment of Fig. 3 were challenged again 6 days later. In Fig. 4, - o - stands for IGELa2-transplanted twice challenged group (n=6), - □ - for untransplanted twice challenged group (n=6), - ● - for IGELa2-transplanted once challenged group, - ■ - for untransplanted once challenged group.

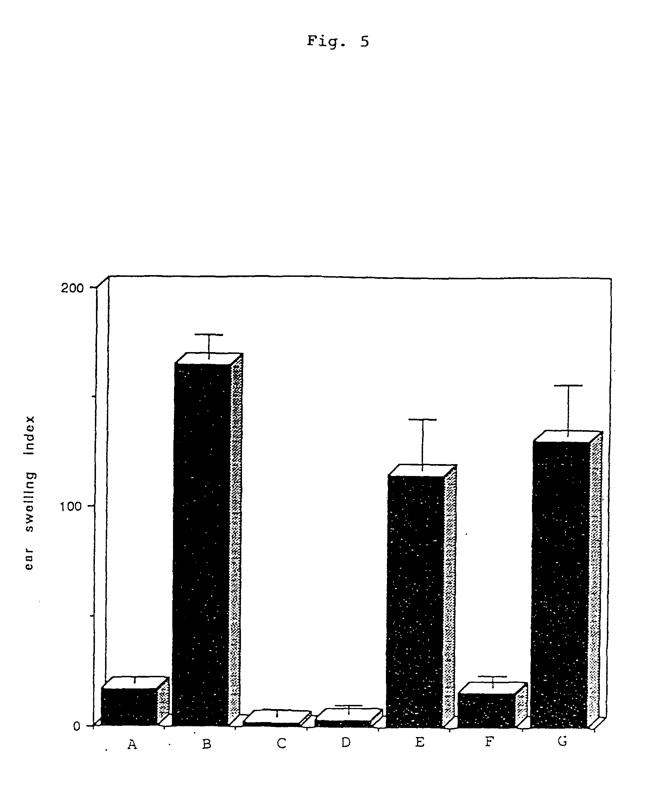

Fig. 5 shows the results of ear swelling in anti-TNP•IgE-producing hybridoma or non-producing hybridoma-transplanted mice. In Fig. 5, column A stands for untransplanted group (n=12), B for a2-transplanted group (n=12), C for a2.1-transplanted group (n=6), D for a2.3-transplanted group (n=6), E for a2.4-transplanted group (n=6), F for a2.15-transplanted mice (n=6) and G for a2.16-transplanted group (n=6).

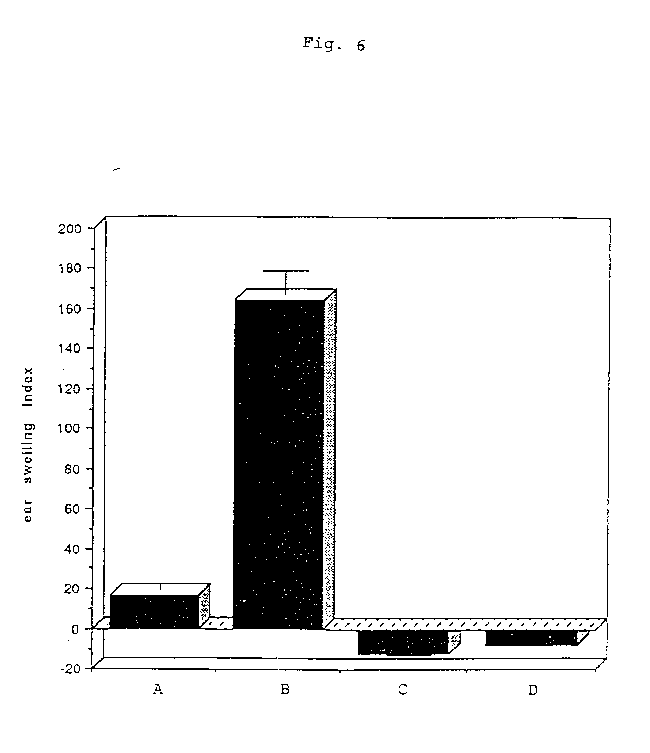

Fig. 6 shows the results of ear swelling in various antigen-challenged anti-TNP•IgE-producing hybridoma IGELa2-transplanted mice. In Fig. 6, column A stands for 0.4% PC-challenged untransplanted group (n=12), column B for 0.4% PC-challenged transplanted group (n=12), column C for OX-challenged transplanted group (n=6) and column D for FITC-challenged transplanted group (n=6).

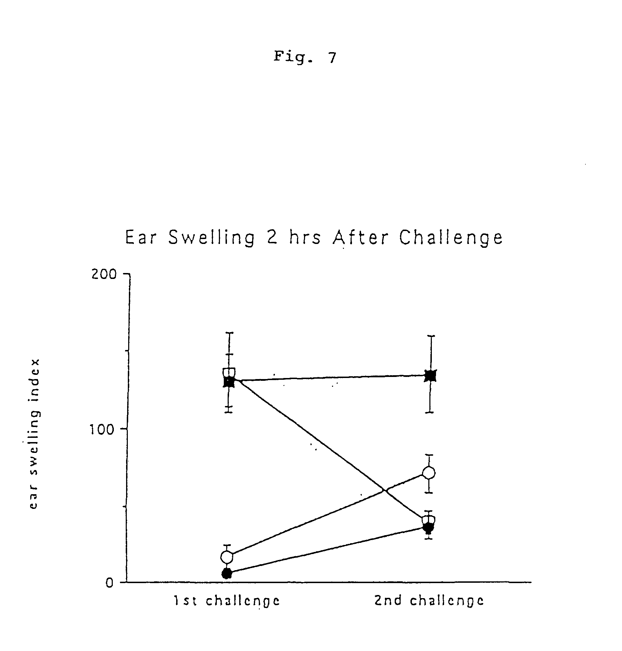

Fig. 7 shows the results of ear swelling of control mice and mice to which human sFcεRIα has been administered after 2 hours of first and second antigen challenges. In Fig. 7, - □ - stands for sFcεRIα-treated IGELa2-transplanted group, - o - for sFcεRIα-treated untransplanted group, - ■ - for untreated IGELa2-transplanted group and - ● - for untreated untransplanted group.

### DETAILED DESCRIPTION OF THE INVENTION

Rodents, such as mouse, rat, guinea pig, or rabbit are employed in the present invention. Particularly, mouse is preferably used.

The term "take" means successful transplantation, namely, that transplanted cells are not rejected by the recipient animal and remain viable in its body.

The IgE-producing vegetative cell is selected from the group of hybridomas consisting of IGELa2 (ATCC No. TIB142), IGELb4 (ATCC No. TIB141) and SE-1.3 (ATCC No. HB137) and the myeloma U266B1 (ATCC No. TIB196).

The means for constructing the animal model is not critical but the following procedures are recommended.

First, the method comprising transplanting a suspension of an IgE-producing hybridoma or myeloma in a syngeneic animal may be mentioned. For example, the procedure of transplanting IGELa2 hybridoma (anti 2,4,6-trinitrophenyl) (ATCC No. TIB142) in BALB/c mice, IGELb4 hybridoma (anti 2,4,6-trinitrophenyl) (ATCC No. TIB141) in (C57BL/6 X BALB/c)F₁ mice or (BALB/c X C57BL/6)F₁ mice, or SE-1.3 hybridoma (anti phenylarsonate) (ATCC No. HB137) in (A/J X BALB/c)F₁ mice or (BALB/c X A/J)F₁ mice may be mentioned.

Secondly, the method comprising transplanting an IgE-producing hybridoma or myeloma in a congenitally immunodeficient animal may be mentioned. Transplantation into nude mice or SCID mice is a typical example.

Thirdly, the method comprising transplanting an IgE-producing hybridoma or myeloma in an animal artificially rendered immunodeficient by X-ray or other treatment may be mentioned.

The method of transplantation is not particularly limited, but generally, the cells to be transplanted are suspended in Hanks' solution, PBS and the like and the resulting suspension is injected at the transplantation site using a syringe.

The site of transplanting an IgE-producing hybridoma or myeloma is not critical but the cells are preferably inoculated subcutaneously or intraperitoneally.

The inoculum size of an IgE-producing hybridoma or myeloma is generally 10³-10¹⁰ and preferably 10⁵-10⁸ cells suspended in 10 to 1,000 µl of Hanks' solution and the like.

In the animal model according to the invention, a remarkable allergy-like symptom can be repeatedly induced by performing an antigen challenge after 6 to 10 days, preferably 7 to 9 days of transplantation of the IgE-producing cells.

The antigen challenge can be carried out by a known method conventionally employed, for example, using an antigen to IgE produced by the vegetative cells, such as picryl chloride. Various antigens other than picryl chloride can be used depending on the site of challenge and the transplanted cells as exemplified below.

| Transplanted cells | Site of challenge | Antigen |
|---|---|---|
| IGELa2 | skin (e.g., auricle) | TNP-HSA |
| | eye | TNP-lysine |
| | digestive tract | TNP-HSA |
| SE-1.3 | skin (e.g., auricle) | p-azobenzenarsonate-L-tyrosine-t-Boc-n-succinimide ester |
| | digestive tract | arsanilated HSA |
| U266B1 | skin (e.g., auricle) | anti-human IgE antigen |

The animal into which the vegetative cells have been transplanted can be fed in the same manner as prior to transplantation.

Screening of prophylactic and therapeutic agents for IgE-related diseases using the animal according to the present invention is carried out by checking as an index as to whether allergy-like symptom induced by the antigen challenge is inhibited by administration of the candidate prophylactic and therapeutic agents or not. Specifically, the antigen challenge induces ear edema when directed to the ear, conjunctivitis-like symptoms when directed to the eye, or rhinitis-like symptoms when directed to the nasal cavity, thus allowing a screening to be carried out using the inhibition of any of such responses as the index.

The prophylactic and therapeutic agents for IgE-related diseases to be screened out using the animal according to the present invention include compositions or compounds useful for preventing and treating IgE-related type I allergic diseases such as bronchial asthma, allergic conjunctivitis, allergic rhinitis, pollen allergy, atopic dermatitis, urticaria and allergic gastroenteritis.

Screening of prophylactic and therapeutic agents for IgE-related diseases can be efficiently and expediently carried out by effecting the antigen challenge repeatedly using the animal model according to the present invention to induce IgE-mediated allergic reaction repeatedly.

The present invention will be described in more detail with reference to the following Example and Test Examples, but is not to be limited thereto.

### EXAMPLE

### Construction of the animal model

Female BALB/c mice (aged 8 weeks or older) were inoculated with 10⁶ cells of the anti-TNP•IgE-producing hybridoma IGELa2 (ATCC No. TIB142) subcutaneously at the back.

### TEST EXAMPLE 1

### Determination of anti-TNP•IgE level in mouse blood

The anti-TNP•IgE-producing hybridoma IGELa2 (ATCC No. TIB142) was rinsed with Hanks' solution and suspended in the same solution. The resulting suspension (10⁶ cells/200 µl) was transplanted subcutaneously at the back of female BALB/c mice (aged 8 weeks or older) using a syringe. (Transplantation was carried out in the same manner in the following Test Examples.) The blood anti-TNP•IgE level of the treated mice was determined by EIA with the passage of time. EIA was carried out using a TNP-HSA-coated plate (10 µg/ml, 50 µl/well) as follows.

The test serum was diluted 30- to 3750-fold with 1% BSA/TBS and 50 µl aliquots were distributed to the plate and incubated at 4°C overnight. The plate was then washed with 0.9% NaCl/0.05% Tween 20 and the bound anti-TNP•IgE was detected using POD-labelled sheep anti-mouse IgE (1:1000, The Binding Site Ltd.) and 1 mg/ml o-phenylenediamine/0.006% H₂O₂. As the purified IgE standard, the IgE purified from a culture supernatant of anti-TNP•IgE-producing hybridoma IGELb4 (ATCC No. TIB141) was used. As a control, no hybridoma-transplanted mice were tested in the same manner as described above.

The results are shown in Fig. 1. The blood anti-TNP•IgE level rose with the passage of time in the hybridoma-transplanted mice but was not elevated in the control mice. A similar result was obtained in mice inoculated with 10⁷ cells of the hybridoma.

### TEST EXAMPLE 2

### Determination of the time course of ear swelling in antigen-challenged mice (I)

Mice were inoculated with 10⁶ IGELa2 cells subcutaneously at the back in the same manner as in Test Example 1. On day 8 after inoculation, 10 µl of 4% picryl chloride in acetone was applied to the left ear of the mice and untreated mice (antigen challenge) and the time course of ear swelling was determined. The difference between the thickness of the ear before the challenge and that after the challenge was used as the index. Determinations were made using dialmeter gauge PEACOCK available from Ozaki Seisakusho.

The results are shown in Fig. 2. Whereas ear swelling became maximal in 1-2 hours after antigen challenge in the hybridoma-transplanted mice, no swelling was observed within 1-2 hours after antigen challenge in the unoperated mice. In mice inoculated with 10⁷ hybridoma cells, ear swelling also became maximal in 1-2 hours after antigen challenge.

The above finding of the maximal ear swelling occurring 1-2 hours after antigen challenge in hybridoma-transplanted mice indicated that the efficacy of an antiallergic agent can be estimated from the degree of inhibition of ear swelling during this time period.

### TEST EXAMPLE 3

### Determination of the time course of ear swelling in antigen-challenged mice (II)

As in Test Example 2, mice were inoculated with 10⁶ IGELa2 hybridoma cells and the first antigen challenge was carried out using 0.4% or 4% picryl chloride (PC) solution on day 8 after inoculation. Control mice were also subjected to antigen challenge.

The results are shown in Fig. 3. It was found that ear swelling became maximal in 2 hours after antigen challenge just as in Test Example 2.

Then, on day 6 after the first antigen challenge, the second antigen challenge was performed in the same manner as the first challenge and using the index described in Test Example 2, the time course of ear swelling was investigated.

The results are shown in Fig. 4. In the hybridoma-transplanted mice, ear swelling became maximal in 2 hours just as after the first antigen challenge and the index value was substantially equal to that at 2 hours after the first challenge. In contrast, the control mice showed no swelling of the ear.

The above results indicate that even if the antigen challenge is performed more than once at the same site in hybridoma-transplanted mice, ear swelling may be repeatedly induced with good reproducibility.

The results further indicate that the use of such hybridoma-transplanted mice not only enables confirmation of the preventive and inhibitory effect of antiallergic agents on primary allergic reaction but also confirmation of the preventive and inhibitory effect on allergic reactions due to the second and subsequent exposures to the antigen.

### TEST EXAMPLE 4

### Proof of anti-TNP•IgE-related type I allergy

(1) As in Test Example 1, mice were inoculated with 10⁶ hybridoma cells subcutaneously at the back. As hybridomas, the above-mentioned IGELa2 and its subclones a2.1, a2.3, a2.4, a2.15 and a2.6 were used. Of these hybridomas, IGELa2, a2.4 and a2.16 were anti-TNP•IgE-producers, but neither IGEL a2.1, a2.3 nor a2.15 secreted anti-TNP•IgE.
   Antigen challenge was carried out in the same manner as in Test Example 2 and the index value was determined 2 hours after the challenge.
   The results are shown in Fig. 5. As shown in Fig. 5, ear swelling was observed with the anti-TNP•IgE producers only.
(2) Antigen challenge in 10⁶ IGELa2-transplanted mice was carried out using, respectively, 1% oxazolone (OX) and 1% fluorescein isothiocyanate (FITC) as well as 0.4% picryl chloride (PC).

The results are shown in Fig. 6. As shown in Fig. 6, ear swelling was observed only in challenges with picryl chloride and not found with the other antigens.

From the results obtained in (1) and (2), it was found that ear swelling requires anti-TNP•IgE and a specific antigen which is recognized by IgE and ear swelling in this assay is an anti-TNP•IgE-mediated type I allergic reaction.

The animal model we constructed allows an allergic reaction to be induced more often than once after a single inoculation with hybridoma cells and is very useful for confirming the efficacy of the candidates of a prophylactic and therapeutic agent for IgE-related diseases.

### TEST EXAMPLE 5

### Inhibitory effect of sFcεRIα on secondary allergic reaction

As in Test Example 3, the first antigen challenge was carried out by applying 10 µl of 0.4% picryl chloride-acetone to the left ear of mice on day 8 after hybridoma transplantation and the second antigen challenge was carried out in the same manner on day 14.

On the other hand, sFcεRIα (JP-A-169776) was administered intravenously in a dose of 25 µg/mouse on days 7 and 8 after transplantation and in a dose of 100 µg/mouse on days 9, 11, 13 and 15. PBS was administered to the control group in place of sFcεRIα. The effect of sFcεRIα was evaluated using the index described in Test Example 2.

The results are shown in Fig. 7. At 2 hours after the first antigen challenge, there was no difference between the control group and the human sFcεRIα-treated group. At 2 hours after the second antigen challenge, however, whereas the control mice showed ear swelling in a degree comparable to that after the first antigen challenge, the ear swelling reaction was inhibited in the mice treated with sFcεRIα.

The above findings show that sFcεRIα completely inhibits allergic reactions due to the second exposure and suggests it will do the same following further exposures to the antigen in patients with allergy and is, therefore, a very satisfactory prophylactic drug.

## Claims

1. A method of screening prophylactic and therapeutic agents for IgE-related diseases which comprises subjecting a rodent model, which is constructed by transplanting an IgE-producing vegetative cell selected from the group consisting of IGELa2 (ATCC No. TIB142), IGELb4 (ATCC No. TIB141), SE-1.3 (ATCC No. HB137) and U266B1 (ATCC No. TIB196) in a non-rejecting rodent, to antigen challenge, administering a candidate substance to the rodent and determining an antiallergic effect of the substance.

2. The method of screening prophylactic and therapeutic agents of Claim 1, wherein said rodent is selected from the group consisting of mouse, rat, guinea pig and rabbit.

## Patentansprüche

1. Verfahren zum Durchmustern von prophylaktischen und therapeutischen Mitteln für IgE-abhängige Erkrankungen, das das Unterwerfen eines Nagetiermodells, welches durch das Transplantieren einer IgE-produzierenden vegetativen Zelle, ausgewählt aus der Gruppe, bestehend aus IGELa2 (ATCC Nr. TIB142), IGELb4 (ATCC Nr. TIB141), SE-1.3 (ATCC Nr. HB137 und U266B1 (ATCC Nr. TIB196), in ein nichtabstossendes Nagetier erzeugt wurde, eine Antigenkonfrontation, die Verabreichung einer Kandidatensubstanz an das Nagetier und das Bestimmen einer antiallergischen Wirkung der Substanz umfasst.

2. Verfahren zum Durchmustern prophylaktischer und therapeutischer Mittel gemäss Anspruch 1, wobei das Nagetier aus der Gruppe ausgewählt ist, die aus Maus, Ratte, Meerschweinchen und Kaninchen besteht.

## Revendications

1. Procédé pour le criblage des agents prophylactiques et thérapeutiques des affections liées aux IgE qui comprend le fait de soumettre un modèle de rongeur, qui est construit en transplantant une cellule végétative produisant des IgE choisie dans le groupe comprenant IGELa2 (ATCC n° TIB142), IGELb4 (ATCC n° TIB141), SE-1.3 (ATCC n° HB137) et U266B1 (ATCC n° TIB196) chez un rongeur ne faisant pas de rejet, à une provocation antigénique, en administrant une substance candidate au rongeur et en déterminant un effet antiallergique de la substance.

2. Procédé pour le criblage des agents prophylactiques et thérapeutiques selon la revendication 1, dans lequel ledit rongeur est choisi dans le groupe comprenant la souris, le rat, le cobaye et le lapin.
